# EUROPEAN PATENT APPLICATION

(11) **EP 2 910 239 A1**
(43) Date of publication of application: **26.08.2015**
(21) Application number: 14382057.9
(22) Date of filing: 20.02.2014
(51) Int. Cl.: A61K 9/127, A61K 35/76, A61K 47/24, A23K 1/00

(54) **Novel compositions comprising lipidic coated bacteriophages**

(71) Applicant: Universitat Autònoma de Barcelona, 08193 Cerdanyola del Vallès (ES); Fundacio Privada Institut Catala de Nanociencia I Nanotecnologia (ICN2), 08193 Bellaterra (Barcelona) (ES); Institució Catalana De Recerca I Estudis Avançats (ICREA), 08010 Barcelona (ES)
(72) Inventor: Maspoch Comamala, Daniel, 08172 Sant Cugat del Vallès (ES); Cano Sarabia, Antonia María, 30560 Alguazas - Murcia (ES); Llagostera Casas, Montserrat, 08290 Cerdanyola del Vallès (España) (ES); Cortés Garmendia, María Pilar, 08010 Barcelona (ES); Campoy Sánchez, Susana, 08221 Terrasa (ES); Colom Comas, Joan, 08570 Torelló (ES)
(74) Representative: Carlos Hernando, Borja

(57) **Abstract**

The present invention provides novel coated bacteriophages and formulations comprising the same, which are characterized by comprising lipidic coated bacteriophages useful in different industrial applications, such as, therapeutic and prophylactic agents in the field of human and veterinary industries, control of bacteria population in livestock in general, and as sanitizing and/or sterilization agents in animal and food production and processing industries. The lipid mixture forming the lipidic capsules, vesicles, envelopes or liposome like particles of the present invention comprises a mixture of four different lipidic components, namely, a first, a second, a third and a fourth lipidic component. Each of these different four lipid components may comprise at least one lipid substance selected from a specific closed list of lipid substances.

SEQUENCE LISTING

<110> Universitat Autonoma de Barcelona
      FUNDACIO PRIVADA INSTITUT CATALA DE NANOCIENCIA I NANOTECNOL
      OGIA (ICN2)
      Institucio Catalana de Recerca i Estudis Avancats ICREA

<120> NOVEL COMPOSITIONS COMPRISING LIPIDIC COATED BACTERIOPHAGES

<130> AX140030EP

<140> EP14382057.9
<141> 2014-02-20

<160> 6

<170> BiSSAP 1.0

<210> 1
<211> 24
<212> DNA
<213> Enterobacteria phage Phi20

<220>
<221> source
<222> 1..24
<223> /mol_type="DNA"
      /note="TTAGATGAACGAAATTTTGGTGGT"
      /organism="Enterobacteria phage Phi20"

<400> 1
ttagatgaac gaaattttgg tggt                                              24


<210> 2
<211> 19
<212> DNA
<213> Enterobacteria phage Phi20

<220>
<221> source
<222> 1..19
<223> /mol_type="DNA"
      /note="CCGAAATGGAATGGTCTGG"
      /organism="Enterobacteria phage Phi20"

<400> 2
ccgaaatgga atggtctgg                                                    19


<210> 3
<211> 23
<212> DNA
<213> Enterobacteria phage UAB_Phi78

<220>
<221> source
<222> 1..23
<223> /mol_type="DNA"
      /note="TATGGCTGGCAAATCTAAGGAGT"
      /organism="Enterobacteria phage UAB_Phi78"

<400> 3

tatggctggc aaatctaagg agt                                              23

<210> 4
<211> 24
<212> DNA
<213> Enterobacteria phage UAB_Phi78

<220>
<221> source
<222> 1..24
<223> /mol_type="DNA"
      /note="ACCACCAAAATTTCGTTCATCTAA"
      /organism="Enterobacteria phage UAB_Phi78"

<400> 4
accaccaaaa tttcgttcat ctaa                                             24

<210> 5
<211> 24
<212> DNA
<213> Enterobacteriaphage UAB_Phi87

<220>
<221> source
<222> 1..24
<223> /mol_type="other DNA"
      /note="CAAAACTTCCTCTTCATCCGTATC"
      /organism="Enterobacteriaphage UAB_Phi87"

<400> 5
caaaacttcc tcttcatccg tatc                                             24

<210> 6
<211> 21
<212> DNA
<213> Enterobacteriaphage UAB_Phi87

<220>
<221> source
<222> 1..21
<223> /mol_type="other DNA"
      /note="GCTGCTGGCATTCTCCCTATC"
      /organism="Enterobacteriaphage UAB_Phi87"

<400> 6
gctgctggca ttctccctat c                                                21

## Description

### FIELD OF THE INVENTION

The present invention relates to the field of bacteriophages, and their application as therapeutic and prophylactic agents in the field of human and veterinary industries, control of bacteriophage populations in livestock in general, as well as sanitizer and sterilization solutions for solid matrices, including food production and processing facilities. In particular, the present invention provides new coated bacteriophages and formulations comprising the same for the said purposes. The new coated bacteriophages are characterized by forming lipid coatings, vesicles, envelopes, capsules or liposome like particles containing in its interior at least one bacteriophage.

The new coated bacteriophages and formulations comprising the same present enhanced protection of the bacteriophages and an improved stability of the formulations, thus providing an increased efficacy during its use.

### BACKGROUND PRIOR ART

It is well known in the state of the art, that bacteriophage preparations can be invaluable in specifically eliminating, or significantly reducing the levels of bacteria in specific environmental settings, such as: environmental clean-up of food processing plants, which would substantially reduce the risk of foodborne pathogens contaminating the food supply, such as slaughterhouses, hospital sanitation, to reduce nosocomial infections caused by pathogenic bacteria, and workplace or equipment decontamination, etc.

Further, the use of bacteriophage in therapy as a treatment option for gastrointestinal pathogenic bacterial infections both in humans and animals has been subject to extensive experimental work in the last decades.

In this context, it is well known in the state of the art that the four main food borne pathogens from animal origin are *Escherichia coli, Campylobacter, Salmonella* and *Listeria.* These bacteria are all common contaminants of ruminants, poultry and swine and are usually carried in their gastrointestinal tract asymptomatically.

Research on the use of bacteriophages against food borne pathogens from animal origin has mainly focused on the optimization of preharvest interventions where the bacteriophage, administration routes and delivery processes have received most attention. Preharvest treatment strategy is primarily directed to administering bacteriophages to livestock to prevent bacterial colonization and/or also to minimize the pathogen carriage in the gastrointestinal tract, thereby preventing pathogen entry to the food supply.

Several different approaches have been used so far, consisting in aerosol formulations, spraying and intramuscular injections, bacteriophage administration via addition to bird drinking water and in general oral delivery systems.

One of the major problems encountered in designing an efficient bacteriophage formulation is providing the necessary protection to the bacteriophage to resist the gastric acidic environment, loss of activity due to low pH conditions and loss of stabilization of the bacteriophage formulation when the bacteriophage formulation is subject to conservation and preservation methods, such as lyophilization.

Different attempts have been made in order to solve the said problems but bacteriophage inactivation is still reported to be the main cause of failure.

The paper "Novel Polymer Matrices for Microencapsulation of Phages: Enhanced Protection Against Acidity and Protease activity", Castro el al., 2012, Macromolecular Bioscience, vol.12, 9, pp1200-1208, describes microencapsulation of bacteriophages in polymeric structures made of pectin and alginate.

"Oral Delivery Systems for Encapsulated Bacteriophages Targeted at Escherichia coli O157:H in Feedlot Cattle", Stanford et al., Journal of Food protection, Vol.73, no.7, 2010, pp. 1304-1312, refers to encapsulation of bacteriophages in Eudragit S100, based on methacrylic and methylmethacrylic acid copolymers.

Canadian patent, CA2463827, describes methods and compositions for controlled release of bioactive compounds, such as bacteriophages, comprising a lyophilized formulation of a bacteriophage comprising a methacrylic acid polymer in addition to lyoprotectants, such as glucose and sucrose.

International application, WO 03/093462, describes immobilization of a bacteriophage on a substrate containing on top thereof a trehalose film.

United States patent application, US20112/0258175, describes a bacteriophage formulation containing stearic and palmitic acid.

The paper, "A lipid nanovesicle system encasing bacteriophages for inhalational therapy", Balcao et al., 2010, XVIII International Conference on Bioencapsulation, Porto, Portugal, October, 1-2, 2010, describes formulating bacteriophages in an oil in water emulsion comprising glycerol, Softisan® (Caprylic/Capric/Myristic/Stearic Triglyceride mixture) and phosphatidylcholine.

Finally, "Microencapsulation of Bacteriophage Felix O1 into Chitosan-Alginate Microspheres for Oral Delivery", Sabour et al, Applied and Enviromental Microbiology, Aug.2008, pp.4799-4805, describes microencapsulation of bacteriophages into chitosan-alginate microspheres for oral delivery.

However, there is still a need for further technical improvement in the field of the present invention, to increase bacteriophage survival during its passage through the animal and human digestive systems for an effective prophylactic use or treatment of intestinal bacterial pathogens, as well as to enhance stability of the formulation during its use in food production and processing industries as well as when the formulations are subject to conventional preservation methods.

The present invention is therefore directed to provide a novel coated bacteriophages and formulations containing them which solve the stated technical problems encountered in the state of the art, procuring enhanced stability properties to the bacteriophages and formulations containing them so as to improve their effectiveness during use thereof.

### SUMMARY OF THE INVENTION

The term "bacteriophage" in the context of the present invention refers to any virus that infects prokaryotic cells.

The terms "cocktail" and "bacteriophage cocktail" in the context of the present invention are equivalent and can be used without distinction. They refer to any formulation containing at least one or more than one bacteriophage, belonging to a different, species, subspecies and/or strain, in a similar or different proportion. Those cocktails additionally can also contain other lytic bacteriophages and/or parts and/or products of them.

The terms, "capsule, vesicle, envelope, and liposome-like particle" in the context of the present invention are equivalent and can be used without distinction. They are meant to include a mixture of amphipathic lipid substances comprising a polar (hydrophilic) headgroup region covalently linked to one or two non-polar (hydrophobic) acyl chains. In presence of aqueous medium, amphipathic lipid substances self-assemble into spherical close structures entrapping part of the aqueous medium in their inner.

The term "lipidic component" in the context of the present invention is meant to include a lipid substance or a mixture of lipid substances formed by one or more lipid substances selected from a defined group or closed list of lipid substances.

The term " a first lipidic component" in the context of the present invention is meant to include at least one lipid substance selected from a first group or closed list of lipid substances, thus including from a single lipid substance to a mixture of lipid substances, all of them selected from a defined first group of lipid substances.

The term "a second lipidic component" in the context of the present invention is meant to include at least one lipid substance selected from a second group or closed list of lipid substances, thus including from a single lipid substance to a mixture of lipid substances, all of them selected from a defined second group of lipid substances.

The term "a third lipidic component" in the context of the present invention is meant to include at least one lipid substance selected from a third group or closed list of lipid substances, thus including from a single lipid substance to a mixture of lipid substances, all of them selected from a defined third group of lipid substances.

The term "a fourth lipidic component" in the context of the present invention is meant to include at least one lipid substance selected from a fourth group or closed list of lipid substances, thus including from a single lipid substance to a mixture of lipid substances, all of them selected from a defined fourth group of lipid substances.

The term "lipid substance" in the context of the present invention relates to each specific lipid substance forming each of the first, second, third and fourth group or closed list of lipid substances.

Lipid substances belonging to the first group or closed list are: 1,2-dilauroyl-rac-glycero-3-phosphocholine (DLPC), 1-palmitoyl-2-oleoyl-sn-glycero-3-phosphocholine (PC), 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC) and 1-palmitoyl-2-oleoyl-sn-glycero-3-phosphocholine (POPC).

Lipid substances belonging to the second group or closed list are: 3β-N-(dimethylaminoethyl) carbamate hydrochloride (cholesteryl), (N-[1-(2,3-Dioleoyloxy)propyl]-N,N,N-trimethylammonium chloride) (DOTAP) and cholesteryl hemisuccinate.

Lipid substances belonging to the third group or closed list are: cholesterol, squalene, ergosterol and phytosterol.

Lipid substances belonging to the fourth group or closed list are: cholesteryl-polyethylene glycol 600 sebacate (Chol-PEG600),1,2-distearoyl-sn-glycero-3-phosphoethanolamine-N-[amino(polyethylene glycol) -2000 (DSPE- PEG2000), N-(Carbonyl-methoxypolyethyleneglycol 2000)-1,2-dipalmitoyl-sn-glycero-3-phosphoethanolamine (DPPE-PEG5000), 1,2-dipalmitoyl-sn-glycero-3-phosphoethanolamine-N-[methoxy(polyethylene glycol)-350] (PE-PEG350), 1,2-dipalmitoyl-sn-glycero-3-phosphoethanolamine-N-[methoxy(polyethylene glycol)-550] (PE-PEG550), 1,2-dioleoyl-sn-glycero-3-phosphoethanolamine-N-[methoxy(polyethylene glycol)-750] (PE-PEG750), 1,2-dimyristoyl-sn-glycero-3-phosphoethanolamine-N-[methoxy(polyethylene glycol)-1000] (PE-PEG1000), 1,2-distearoyl-sn-glycero-3-phosphoethanolamine-N-[methoxy(polyethylene glycol)-2000] (PE-PEG2000), 1,2-dimyristoyl-sn-glycero-3-phosphoethanolamine-N-[methoxy(polyethylene glycol)-3000] (PE-PEG3000) and 1,2-dioleoyl-sn-glycero-3-phosphoethanolamine-N-[methoxy(polyethylene glycol)-5000] (PE-PEG5000).

The term "animal" in the context of the present invention is meant to include any warm-blooded animal, included humans. In a preferred embodiment animals are selected from the group consisting of *Gallus gallus,* turkeys and other avian species, in addition to pigs and cattle.

The term "food" in the context of the present invention is meant to include any plant, animal or any substance or material from plants and animals (vegetables, meat, eggs, etc.) that provides nutritional support for the life of humans and animals.

The term "solid matrices" in the context of the present invention is meant to include any surface which may be colonized by a microorganism to be eliminated. Those solid matrices include animal skin, surfaces of farms, slaughterhouses, poultry and swine barns and/or pens, crates used during animal transportation from farms to the food processing facilities and other facilities used during animal and food manufacturing and processing.

The term "acceptable carrier, vehicles, solvents and/or excipients" in the context of the present invention are meant to include any carrier, whether pharmaceutically acceptable or not, which does not cause significant irritation to an organism and does not abrogate the biological activity and properties of the administered compound/composition. These vehicles include those acceptable and suitable for administering the compound/composition for an intended prophylactic and/or therapeutic treatment and those acceptable and suitable as feed additives or sanitizer and sterilization solutions. Examples include: water-based carriers, saline water, sterile water, Ringer's solution, buffered physiological saline, etc.

According to a first aspect, the invention relates to new coated bacteriophages and formulations containing them, comprising a lipid mixture encasing, enclosing or enveloping one or more bacteriophages and forming a lipidic capsule, vesicle, envelope or liposome-like particle containing one or more bacteriophages characterized in that the lipid mixture comprises:
- a first lipidic component containing at least one lipid substance selected from the group consisting of: 1,2-dilauroyl-rac-glycero-3-phosphocholine (DLPC), 1-palmitoyl-2-oleoyl-sn-glycero-3-phosphocholine(PC), 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC) and 1-palmitoyl-2-oleoyl-sn-glycero-3-phosphocholine (POPC),
- a second lipidic component containing at least one lipid substance selected from the group consisting of: 3β-N-(dimethylaminoethyl) carbamate hydrochloride (cholesteryl), (N-[1-(2,3-Dioleoyloxy)propyl]-N,N,N-trimethylammonium chloride) (DOTAP) and cholesteryl hemisuccinate,
- a third lipidic component containing at least one lipid substance selected from the group consisting of: cholesterol, squalene, ergosterol and phytosterol,
   and
- a fourth lipidic component containing at least one lipid substance selected from the group consisting of: cholesteryl-polyethylene glycol 600 sebacate (Chol-PEG600),1,2-distearoyl-sn-glycero-3-phosphoethanolamine-N-[amino(polyethylene glycol)-2000 (DSPE- PEG2000), N-(Carbonyl-methoxypolyethyleneglycol 2000)-1,2-dipalmitoyl-sn-glycero-3-phosphoethanolamine (DPPE-PEG5000), 1,2-dipalmitoyl-sn-glycero-3-phosphoethanolamine-N-[methoxy(polyethylene glycol)-350] (PE-PEG350), 1,2-dipalmitoyl-sn-glycero-3-phosphoethanolamine-N-[methoxy(polyethyleneglycol)-550] (PE-PEG550), 1,2-dioleoyl-sn-glycero-3-phosphoethanolamine-N-[methoxy(polyethylene glycol)-750] (PE-PEG750), 1,2-dimyristoyl-sn-glycero-3-phosphoethanolamine-N-[methoxy(polyethylene glycol)-1000] (PE-PEG1000), 1,2-distearoyl-sn-glycero-3-phosphoethanolamine-N-[methoxy(polyethylene glycol)-2000] (PE-PEG2000), 1,2-dimyristoyl-sn-glycero-3-phosphoethanolamine-N-[methoxy(polyethylene glycol)-3000] (PE-PEG3000) and 1,2-dioleoyl-sn-glycero-3-phosphoethanolamine-N-[methoxy(polyethylene glycol)-5000] (PE-PEG5000).

A further aspect of the present invention is directed to coated bacteriophages and formulations containing them, characterized in that the lipid mixture forming the lipidic capsules, vesicles, envelopes or liposome like particles comprises:
- the said first lipidic component in an amount ranging from 40-60% weight,
- the said second lipidic component in an amount ranging from 30-45% weight,
- the said third lipidic component in an amount ranging from 10-40% weight, and
- the said fourth lipidic component in an amount ranging from 0.5-10% weight.

A preferred object of the present invention is directed to coated bacteriophages and formulations containing them, characterized in that the lipid mixture forming the lipidic capsules, vesicles, envelopes or liposome-like particles comprises:
- the first lipidic component in an amount of 50% weight,
- the second lipidic component in an amount of 35% weight,
- the third lipidic component in an amount of 10% weight, and
- the fourth lipidic component in an amount of 5% weight.

A more preferred object of the present invention is directed to coated bacteriophages and formulations containing them, characterized in that the lipid mixture forming the lipidic capsules, vesicles, envelopes or liposome-like particles comprises:
- 50% weight 1,2-dilauroyl-rac-glycero-3-phosphocholine (DLPC),
- 35% cholesteryl 3P-N-(dimethylaminoethyl)carbamate hydrochloride (cholesteryl),
- 10% cholesterol (Chol), and
- 5% cholesteryl-polyethylene glycol 600 sebacate (Chol-PEG600).

A still further object of the present invention is directed to coated bacteriophages as previously defined and formulations containing them, characterized in that the one or more bacteriophages are selected from the group consisting of: tailed bacteriophages from the *Caudovirales* order, UAB_Phi20, deposited in GenBank under accession number GQ422450, (version: GQ422450.1 GI: 321282844), UAB_Phi78 deposited in GenBank under accession number GU595417 (version GU595417.1 GI: 322227495) and UAB_Phi87, deposited in GenBank under accession JN225449, (version JN225449.1 GI: 402760815).

And a still preferred object of the present invention is directed to coated bacteriophages as previously defined and formulations containing them, characterized in that the one or more bacteriophages are selected from the group consisting of: UAB_Phi20, deposited in GenBank under accession number GQ422450, (version: GQ422450.1 GI: 321282844), UAB_Phi78 deposited in GenBank under accession number GU595417 (version GU595417.1 GI: 322227495) and UAB_Phi87, deposited in GenBank under accession JN225449, (version JN225449.1 GI: 402760815), and in that the lipid mixture forming the lipidic capsules, vesicles, envelopes or liposome-like particles is consisting of:
- 50% weight 1,2-dilauroyl-rac-glycero-3-phosphocholine (DLPC),
- 35% cholesteryl 3P-N-(dimethylaminoethyl)carbamate hydrochloride (cholesteryl),
- 10% cholesterol (Chol), and
- 5% cholesteryl-polyethylene glycol 600 sebacate (Chol-PEG600).

A further object of the present invention is directed to coated bacteriophages and formulations comprising the same, as previously defined, which are lyophilized.

Further objects of the present invention are directed to the said coated bacteriophages and formulations comprising the same characterized in that said coated bacteriophages have a mean particle size of between 200 - 600 nm, and/or a net surface charge of +10/+50mV, and/or are small unilamellar vesicles.

An additional object of the present invention is directed to coated bacteriophages and formulations comprising the same, according to the said previous features for the prophylactic and therapeutic treatment of bacterial infections in humans and animals, as feed additives or for sanitization and/or sterilization of solid matrices in food production and processing facilities, in particular to control *Salmonella* populations in food and solid matrices.

A still additional object of the present invention is a bacteriophage formulation comprising coated bacteriophages previously defined together with acceptable carriers, vehicles, solvents and/or excipients for the said intended purposes.

A still further object of the present invention is a bacteriophage formulation comprising between 40% to 55% of coated bacteriophages according to the present invention versus total titre of bacteriophages.

A further object of the present invention are bacteriophage formulations as defined previously in the form of suspensions, sprays, aerosols, powders or granules, emulsions, hard or soft capsules, syrups or elixirs.

A further object of the present invention is bacteriophage formulations as defined previously in the form of an animal feed, drinking water, sanitizer or sterilization solution.

A still further object of the present invention is directed to a process for obtaining the coated bacteriophages according to the present invention, comprising coating the bacteriophages with a lipid mixture by hydrating under agitation an aqueous solution containing the desired bacteriophages, at a concentration up to 10¹¹ pfu/mL, with a dry lipid film having a total amount of lipids from 10 to 30 mM.

And finally it is also an object of the present invention a process for obtaining coated bacteriophages which are lyophilized, comprising coating the bacteriophages with a lipid mixture by hydrating under agitation an aqueous solution containing the desired bacteriophages at a concentration up to 10¹¹ pfu/mL and a cryoprotective agent, in particular, trehalose with a dry lipid film having a total amount of lipids from 10 to 30 mM.

### FIGURES

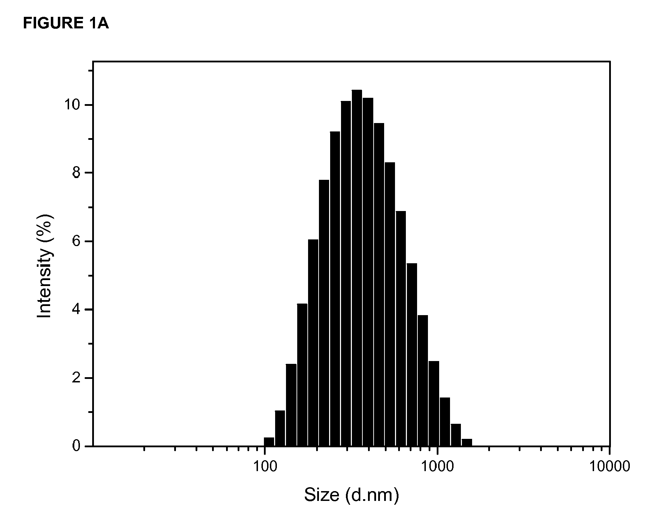

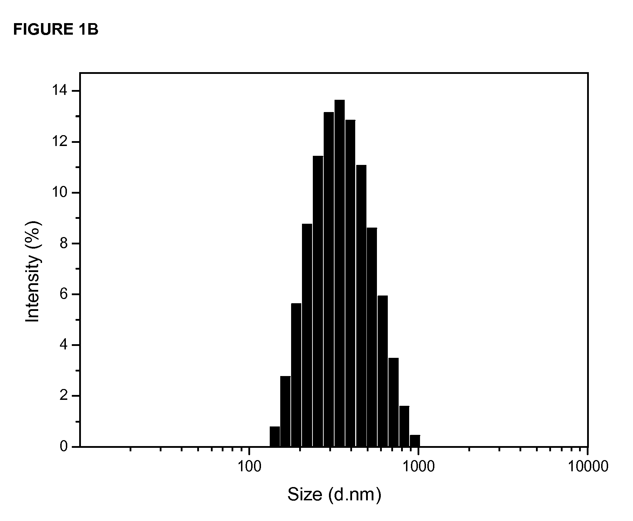

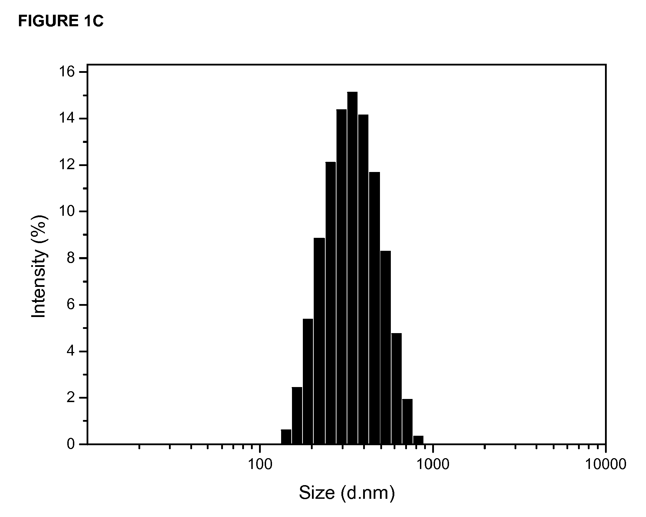

Figures 1A - C represent the mean size and particle size distribution of lipidic coated bacteriophages, Fig. 1A: lipidic coated bacteriophage UAB_Phi78, Fig. 1B: lipidic coated bacteriophage UAB_Phi87 and Fig. 1C: lipidic coated bacteriophage UAB_Phi20.

### DETAILED DESCRIPTION OF THE INVENTION

The present invention provides novel coated bacteriophages and formulations comprising the same, which are characterized by comprising lipidic coated bacteriophages, in other words, lipidic capsules, vesicles, envelopes or liposome-like particles comprising one or more bacteriophages in each capsule, vesicle, envelope or liposome-like particle.

These novel coated bacteriophage formulations are useful in different industrial applications, such as, therapeutic and prophylactic agents in the field of human and veterinary industries, control of bacteria population in livestock in general, and as sanitizing and/or sterilization agents in animal and food production and processing industries.

These novel coated bacteriophage and the formulations comprising them are designed to improve the stability of the bacteriophages, which in turn leads to a substantial improvement in the survival rate of the bacteriophage during administration or use of the coated bacteriophages and the formulations comprising the same.

In particular, and as regards the use of the coated bacteriophages for prophylactic and/or therapeutic treatment of bacterial infections as well as control of bacterial populations in livestock in general, this requires administering the coated bacteriophages and/or the formulations containing them to an animal or human subject and accordingly, efficiency of infectivity depends on as long as possible residence time of the bacteriophages in the digestive tract and intestine where the bacteriophages are expected to exert bacterial infection. As has been proven in the present invention, residence time in the digestive tract and intestine of the coated bacteriophages of the present invention is substantially increased when compared to non-coated bacteriophages.

In addition coating of the bacteriophages of the present invention is intended to provide the necessary protection to the bacteriophages during the encasing or encapsulation of the bacteriophage/s.

Furthermore the lipidic coat designed according to the present invention protects the bacteriophages from getting inactivated by extreme process conditions when such coated bacteriophages or the formulations comprising the same are subject to a preservation method, such as a lyophylization, desiccation or freeze-drying processes.

The coated bacteriophages of the present invention are characterized by comprising a lipid mixture encasing, enclosing or enveloping one or more bacteriophages and thus forming lipidic capsules, vesicles, envelopes and/or liposome like particles containing one or more bacteriophages in each of these lipidic capsules, vesicles, envelopes and/or liposome like particles.

These capsules, vesicles, envelopes or liposome like particles show a mean particle size of between 200-600 nm, preferably 320 nm, and a net surface charge of +10 / +50 mV, preferably, +35 mV.

As regards the shape and form of the lipidic capsules, vesicles, envelopes or liposome like particles of the present invention, these present preferably spherical form and are small unilamellar vesicles (SUVs).

The lipid mixture forming the lipidic capsules, vesicles, envelopes or liposome like particles of the present invention is comprising a mixture of four different lipidic components, namely, a first, a second, a third and a fourth lipidic component. Each of these different four lipid components may comprise at least one lipid substance selected from a specific closed list of lipid substances.

In other words, each of the first, second, third and fourth lipidic components may comprise one, two or more lipid substances selected from a first, a second, a third and a fourth closed list or group of lipid substances.

In a preferred embodiment of the present invention, each of the first, second, third and fourth lipidic component is consisting of one lipid substance selected from each of the first, second, third and fourth closed list of lipid substances.

The lipid substances contained in each group are different lipid substances. In particular, lipid substances belonging to the first closed list and forming part of the first lipid component are: 1,2-dilauroyl-rac-glycero-3-phosphocholine (DLPC), 1-palmitoyl-2-oleoyl-sn-glycero-3-phosphocholine (PC), 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC) and 1-palmitoyl-2-oleoyl-sn-glycero-3-phosphocholine (POPC).

Lipid substances belonging to the second closed list and forming part of the second lipid component are: 3β-N-(dimethylaminoethyl) carbamate hydrochloride (cholesteryl), (N-[1-(2,3-Dioleoyloxy)propyl]-N,N,N-trimethylammonium chloride) (DOTAP) and cholesteryl hemisuccinate.

Lipid substances belonging to the third closed list and forming part of the third lipidic component are: cholesterol, squalene, ergosterol and phytosterol.

Lipid substances belonging to the fourth closed list and forming part of the fourth lipidic component are: cholesteryl-polyethylene glycol 600 sebacate (Chol-PEG600),1,2-distearoyl-*sn*-glycero-3-phosphoethanolamine-N-[amino(polyethylene glycol)-2000 (DSPE- PEG2000), N-(Carbonyl-methoxypolyethyleneglycol 2000)-1,2-dipalmitoyl-sn-glycero-3-phosphoethanolamine (DPPE-PEG5000), 1,2-dipalmitoyl-sn-glycero-3-phosphoethanolamine-N-[methoxy(polyethylene glycol)-350] (PE-PEG350), 1,2-dipalmitoyl-sn-glycero-3-phosphoethanolamine-N-[methoxy(polyethylene glycol)-550] (PE-PEG550), 1,2-dioleoyl-sn-glycero-3-phosphoethanolamine-N-[methoxy(polyethylene glycol)-750] (PE-PEG750), 1,2-dimyristoyl-sn-glycero-3-phosphoethanolamine-N-[methoxy(polyethylene glycol)-1000] (PE-PEG1000), 1,2-distearoyl-sn-glycero-3-phosphoethanolamine-N-[methoxy(polyethylene glycol)-2000] (PE-PEG2000), 1,2-dimyristoyl-sn-glycero-3-phosphoethanolamine-N-[methoxy(polyethylene glycol)-3000] (PE-PEG3000) and 1,2-dioleoyl-sn-glycero-3-phosphoethanolamine-N-[methoxy(polyethylene glycol)-5000] (PE-PEG5000).

Further, preferably, each of the first, second, third and fourth lipidic component is present in the lipidic mixture forming the lipidic capsules, vesicles, envelopes or liposome like particles of the present invention in a defined amount calculated on a % by weight basis, in particular, the first lipidic component is present in an amount ranging from 40-60%.weight, preferably, 50%, the second lipidic component in an amount ranging from 30-45%.weight, preferably 35%, the third lipidic component in an amount ranging from 20-40%.weight, preferably, 10% the fourth lipidic component in an amount ranging from 0.5-1 0%.weight, preferably 5%.

A preferred lipid mixture according to the present invention is represented by the following lipid components in the specified amounts (weight %):
- 50% of 1,2-Dilauroyl-rac-glycero-3-phosphocholine (DLPC),
- 35% of cholesteryl 3P-N-(dimethylaminoethyl)carbamate hydrochloride (cholesteryl),
- 10% of cholesterol (Chol), and
- 5% of cholesteryl-polyethylene glycol 600 sebacate (Chol-PEG600).

The coated bacteriophages according to the present invention, formed by lipidic capsules, vesicles, envelopes or liposome like particles which comprise one or more bacteriophages in each lipid capsule, vesicle, envelope or liposome like particle are obtained by thin film hydration technique.

This method implies the forming of vesicles during hydration and swelling of a dry lipid film in the presence of a solution containing bacteriophages.

The method involves in general terms the following phases: preparation of the dry lipid film for hydration, hydration under agitation in the presence of the solution containing bacteriophages and sizing to a homogeneous distribution of vesicles.

The lipid mixture is prepared by dissolving the desired lipids in an organic solvent to ensure a homogeneous mixture of lipids. The amount of total lipids ranges from 10 to 30 mM, preferably, 17 mM. This process can be carried out using chloroform or chloroform:methanol mixtures. Once the lipids are thoroughly mixed in the organic solvent, the solvent is removed by, for example, evaporation under vacuum, to yield a lipid film. Thereafter, the lipid film is thoroughly dried to remove any residual solvent by using a dry nitrogen or argon stream. Thereafter, coating of bacteriophages proceeds by hydrating the dry lipid film under agitation in an aqueous solution, preferably, an aqueous solution of MgSO₄ 10mM containing the desired bacteriophages, at a concentration up to 10¹¹pfu/mL. The product resulting from hydration is formed by the said lipidic capsules, vesicles, envelopes or liposome like particles containing said bacteriophages.

Once the stable, hydrated, lipidic coated bacteriophages suspension has been produced, the particles are homogenized by extrusion through a, for example, polycarbonate filter with a defined pore size.

An additional feature of the present invention refers to the percentage of bacteriophages which may be coated according to the present invention. As has been shown in Example 3, the percentage of coated bacteriophages versus total titre of bacteriophages is comprised between 40% to 55%.

These capsules, vesicles, envelopes or liposome-like particles show a mean particle size of between 200-600 nm, preferably 320 nm, as measured by dynamic light scattering technique (DLS), as shown in Figures 1A, B and C.

The present invention is directed to coating any bacteriophage or bacteriophage cocktail capable of lysing at least one microorganism, useful in the prophylactic and therapeutic treatment of bacterial infections in humans and animals, as feed additives or for sanitization and/or sterilization of solid matrices in food production and processing facilities, in particular to control *Salmonella* populations in food and solid matrices.

Bacteriophages which may be used for the purpose of the present invention may be selected from lytic bacteriophages useful in human and animal therapy.

Amongst others, a preferred embodiment of the present invention is represented by coated bacteriophages selected from the group consisting of: tailed bacteriophages from the *Caudovirales* order, UAB_Phi20, deposited in GenBank under accession number GQ422450, (version: GQ422450.1 GI: 321282844), UAB_Phi78 deposited in GenBank under accession number GU595417 (version GU595417.1 GI: 322227495) and UAB_Phi87, deposited in GenBank under accession JN225449, (version JN225449.1 GI: 402760815).

The present invention further provides a method for the treatment of infections produced by pathogenic bacteria consisting in administering to the human or animal in need thereof, an effective amount of the coated bacteriophages and/or formulations containing them together with suitable pharmaceutical acceptable vehicles or feed additives.

The present invention further provides a method for controlling bacterial population in livestock, food and any solid matrices, consisting in applying an effective amount of the coated bacteriophages and/or formulations containing them together with suitable acceptable vehicles or additives to the solid matrices or livestock.

More specifically, the present invention is suitable for providing stable and effective coated bacteriophages and formulations comprising the same against bacteria populations, such as, but not limited to, *Bacillus cereus, Bacillus anthracis, Bacillus subtitils, Bacillus thuringiensis, Bacillus stearothermophilus, Vibrio parahemolyticus, Vibrio cholera, Vibrio vulnificus, Salmonella enterica, Clostridium difficile, Clostridium botulinum, Clostridium perfringens, Staphylococcus aureus, Escherichia coli, Campylobacter jejuni, Campylobacter coli, Campylobacter lari, Campylobacter fetus, Yersinia enterocolitica, Yersinia pestis, Yersinia pseudotuberculosis, Listeria monocytogenes, Shigella, Streptococcus, Actinobacillus, Lactobacillus, Citrobacter* and *Pseudomonas aeruginosa.*

Most preferred embodiment of the present invention are coated *Salmonella* specific bacteriophages and/or coated bacteriophage cocktails belonging to the Caudovirales order, selected from the group consisting of UAB_Phi20, UAB_Phi78 and UAB_Phi87, deposited in GenBank under accession numbers GQ422450, GU595417 and JN225449 respectively, and described in international patent application number WO2013014273 characterized in that the lipid mixture forming the envelope, vesicle, capsule or liposome like particle is consisting of the following lipid components in the specified amounts (weight %):
- 50% 1,2-dilauroyl-rac-glycero-3-phosphocholine (DLPC),
- 35% cholesteryl 3β-N-(dimethylaminoethyl)carbamate hydrochloride (cholesteryl),
- 10% cholesterol (Chol), and
- 5% cholesteryl-polyethylene glycol 600 sebacate (Chol-PEG600).

In certain embodiments of the present invention, the coated bacteriophages and formulations comprising the same are suitable for oral administration, injection, inhalation, spraying and immersion.

The coated bacteriophages and formulations comprising the same may be in the form of suspensions, powders, granules, emulsions, hard or soft capsules, syrups or elixirs.

In each of these cases the coated bacteriophages and the formulations comprising the same may optionally contain in addition to the coated bacteriophages, suitable vehicles known from the state of the art, intended for pharmaceutical use, non-pharmaceutical use, such as sterilization and/or sanitizing solutions or feed additives, e.g., solvents, including water-based formulations, pharmaceutically acceptable excipients, saline, sterile water, Ringer's solution, buffered physiological saline, etc.

Either the coated bacteriophages and/or the formulations comprising the same can be lyophilized so as to preserve these products for long-term stability, storage and handling purposes. These freeze dried powders are directly applicable as dry-powder inhalers or are reconstituted for oral administration, injection or nebulization.

The lyophilization process followed in the present invention comprises incorporating a lyoprotective agent, such as, disaccharides, for example, mannitol, sucrose and/or trehalose, preferably trehalose, to the lipid coated phage preparation step. In this case, the dry lipid film was hydrated with a solution containing the bacteriophages, and trehalose at a 1:3.1 - 1:7.8 lipid to carbohydrate ratio (2 - 5% (w/v)), preferably, at a 1:5 lipid to carbohydrate ratio (3.2% (w/v)). Lipid vesicles loaded with bacteriophages and trehalose were frozen in liquid nitrogen and finally lyophilized (48 h at -80 °C) and stored at room temperature.

The stability at 4°C of the lipid coated bacteriophages was evaluated by titration of these bacteriophages at 0, 15, and 30 days post-encapsulation.

Freeze dried coated bacteriophages according to the present invention have been stored at room temperature for at least one month.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are intended for purposes of illustration only and are not intended to limit the scope of protection. Furthermore, the present invention covers all possible combinations of particular and preferred embodiments described herein.

### EXAMPLES

### Example 1: Obtaining bacteriophages

### Bacterial strains and growth conditions

The virulent strains *Salmonella enterica* serovar Typhimurium ATCC14028 (American Type Culture Collection) and *Salmonella enterica* serovar Enteritidis LK5 (*Salmonella* Genetic Stock Centre, University of Calgary) and non-virulent strain S. Typhimurium LB5000 (SGSC181; University of Calgary) were used for the propagation of *Salmonella's* bacteriophages UAB_Phi20, UAB_Phi78 and UAB_Phi87.

### Propagation and purification of bacteriophages

UAB_Phi20, UAB_Phi78 and UAB_Phi87 bacteriohage lysates were obtained by infecting exponential cultures of S. Typhimurium LB5000 grown in LB broth at a multiplicity of infection (MOI; relation between the numbers of infective bacteriophages per bacterial viable cell) of 0.01 pfu/cfu, and incubated at 37°C during 5 h. After the centrifugation of cultures at 10,414 x g for 10 min, the supernatant was collected and filtered through a 0.45 µm and 0.22 µm syringe polyestersulfona (PES) filters and the bacteriophage titter was determined by plating serial dilutions (1:10) onto LB plates using the double agar layered method.

When high titter of bacteriophage lysates were necessary, 50 ml of *Salmonella* cultures in LB broth at an initial O.D.₅₅₀ of 0.2 were prepared and incubated at 37°C with agitation till obtaining O.D.₅₅₀ of 1. Then the culture was infected with the appropriated bacteriophage keeping a MOI of 0.01. After the infection the suspension was incubated at 37°C for 12-14 h. Finally, the lysates were recovered similarly as described previously. The bacteriophage lysates were concentrated and purified by the following method. First of all, the bacteriophages were concentrated by ultracentrifugation (OptimaTM L-80, Beckman, California, USA), in an 80Ti rotor (Beckman, California, USA) for 2 h at 51,000 x g at 4°C. All pellets of the bacteriophages were resuspended with MgSO₄ 10 mM and kept shaking overnight. After that, the suspension was filtered through a 0.45 µm PES filters and stored at 4°C. The bacteriophage titter was determined as above described. The bacteriophage stock solutions were maintained in MgSO₄ 10 mM in milli-Q water solution at 4 °C retaining a constant titter during several months.

### Checking purity of bacteriophage lysates by PCR

To control the purity of the bacteriophages UAB_Phi20, UAB_Phi78 and UAB_Phi87 lysates, a method based in the detection of bacteriophages through conventional PCR on the lysates was used. For this purpose, a pair of specific primers to detect DNA primase genes in the three bacteriophages (UAB_Phi20, UAB_Phi78 and UAB_Phi87) were designed (Table 1). PCR reactions were performed using the Expand High Fidelity mix (Roche) using as DNA template bacteriophage suspensions yielding a titre of 1 x 10⁵ - 1 x 10¹¹ pfu/ml and previously boiled at 100°C for 10 min. Specific primers were used in individual PCR reactions with the following thermal cycling conditions: 95°C for 5 min, 30 cycles at 95°C for 30 s, 57°C for 30 s, and 72°C for 1 min, and a final extension step of 7 min at 72°C.

**Table 1. Primers used for detection of bacteriophages UAB_Phi20, UAB_Phi78 and UAB_Phi87.**

| **Primer name** | **Gene position (bp)** | **Primer sequence** |
|---|---|---|
| UAB_Phi20 GP18 up | 116-139 | 5'-TTAGATGAACGAAATTTTGGTGGT-3' |
| UAB_Phi20 GP18 Rv | 731-713 | 5'-CCGAAATGGAATGGTCTGG-3' |
| UAB_Phi78 GP13 up | 936-958 | 5'-TATGGCTGGCAAATCTAAGGAGT-3' |
| UAB_Phi78 GP13 Rv | 1542-1519 | 5'-ACCACCAAAATTTCGTTCATCTAA-3' |
| UAB_Phi87 ORF131 up | 534-557 | 5'-CAAAACTTCCTCTTCATCCGTATC-3' |
| UAB_Phi87 ORF131 Rv | 1214-1194 | 5'-GCTGCTGGCATTCTCCCTATC-3' |

The table shows the primer sequences and their position in the DNA primase gene of each bacteriophage.

### Example 2: Lipidic coating of bacteriophages UAB_Phi20, UAB_Phi78 and UAB_Phi87

The coating of bacteriophages in lipids was prepared using the thin film hydration method from a lipid mixture of DLPC, Chol-PEG, Chol and cholesteryl at 1:0.1:0.2:0.7 molar ratios respectively. The amount of total lipid was 17 mM. Lipids were dissolved in chloroform and the solvent was removed by evaporation under vacuum and nitrogen. The lipids were hydrated with the appropriate solution of each of the bacteriophages, UAB_Phi20, UAB_Phi78 and UAB_Phi87 at a concentration in each case of 10¹¹ pfu/mL, and the lipid structures thus obtained were homogenized to 400 nm by means of an extruder (Lipex Biomembranes, Vancouver, Canada). Lipid envelopes loaded with the bacteriophages present a mean particle size of 320 nm with a polydispersity index (Pdl) of 0.19, which is an optimal size for an efficient bacteriophage encapsulation. Additionally, zeta potential measurements revealed a net surface charge of +35 mV on liposomes. Liposomes loaded with bacteriophages are close structures and present small unilamellar vesicle (SUVs) morphology.

### Example 3: Titration of lipidic coating bacteriophages

The titre of lipidic coating bacteriophages obtained in the previous example was determined by plating serial dilutions (1:10) onto LB plates using the double agar layered using the same *Salmonella* tester strain. This titre corresponds to the free or non-coated bacteriophages. In order to ascertain the coating efficiency, the total titre of bacteriophages was determined, by treating 0.5 ml of the coated bacteriophages with 0.5 ml of bile salts 50 mM (Sigma-Aldrich). Afterwards, appropriate dilutions were plated using the double agar layer method. The percentage of coating was calculated using the following formula: 100-[(free bacteriophages titre/total titre)x100], obtaining a coating percentage of bacteriophages comprising between 40% to 55%. In this sense, the percentage (%) of lipid coating for the bacteriophages UAB_Phi20, UAB_Phi78 and UAB_Phi87 was of 50 ± 1.1, 43.3 ± 2.3, and 41.6 ± 3.1, respectively, after three independents experiments.

### Example 4. Stability of lipidic coated bacteriophages at 4°C

The effect of the storage temperature of 4°C on infectivity of lipidic coating bacteriophages UAB_Phi20, UAB_Phi78 and UAB_Phi87 of the present invention was determined. For this purpose, coated bacteriophages were kept a 4°C for 30 days. The capsules were tittered at 0, 15 and 30 days. The titration of the coating bacteriophages were done as detailed in Example 3.

The results indicated that the percentage of UAB_Phi20, UAB_Phi78 and UAB_Phi87 bacteriophage lipidic capsules remained stable for 30 days when they are stored at 4°C (Table 2).

**TABLE 2. Stability of bacteriophage lipidic capsules after storage at 4 °C.**

| Bacteriophage | Percentage of coated bacteriophages* | | |
|---|---|---|---|
| | Storage time (days) | | |
| | 0 | 15 | 30 |
| UAB_Phi20 | 50 ± 1.1 | 66.7 ± 0.6 | 45.5 ± 3.5 |
| UAB_Phi78 | 43.3 ± 2.3 | 45 ± 1.4 | 35.5 ± 3.5 |
| UAB_Phi87 | 41.6 ± 3.1 | 56 ± 5.7 | 57 ± 3.1 |

| | | | |
|---|---|---|---|
| * Each value is the average of three independent experiments ± standard error | | | |

### Example 5. Stability of lipidic coated bacteriophages at acidic pH 2.8

The effect of pH on infectivity of lipidic coating bacteriophages UAB_Phi20, UAB_Phi78 and UAB_Phi87 of the present invention was determined by mimicking stomach conditions. For this purpose, a solution composed by NaCl 0.85%; pepsin (Sigma) 3 mg/ml, and pH 2.8 and an incubation temperature of 42 °C was used. The methodology was as followed, 0.1 ml of the lipidic coating bacteriophages were added to 10 ml of the stomach solution while the pH was checked. The bottles were incubated in a water bath with agitation and samples were taken at 0, 30, and 60 min. Those samples were treated with bile salts 50 mM and plated as described previously to determine the lipidic coating bacteriophages total titre. The same methodology was applied to non-coated bacteriophages in order to determine the protection efficacy of the lipid envelopes on the infectivity of the bacteriophages.

The results indicated that lipidic envelopes protect bacteriophages from the acid pH. After 60 min in contact with acid pH, coated bacteriophages showed a titre of 0.9 log¹⁰, 1.7 log¹⁰ and, 2.3 log¹⁰ for UAB_Phi20, UAB_Phi78 and UAB_Phi87, respectively, higher compared with the non-coated bacteriophages (Table 3). Therefore, lipidic coating confers to the bacteriophages a high stability at extremely acid pH.

**TABLE 3. Survival of lipidic coating bacteriophages after exposure to acid pH**

| Bacteriophage | Lipidic coat | Bacteriophage titre (Log10 pfu/ml) | | |
|---|---|---|---|---|
| | | Time exposure (min) | | |
| | | 0 | 30 | 60 |
| UAB_Phi20 | + | 9.2 ± 0.0 | 6.2 ± 0.1 | 4.4 ± 0.3 |
| | - | 9.2 ± 0.1 | 5.4 ± 0.2 | 3.5 ± 0.1 |
| UAB_Phi78 | + | 9.0 ± 0.2 | 4.5 ± 0.4 | 1.7 ± 0.1 |
| | - | 9.3 ± 0.2 | 1.2 ± 0.2 | ND |
| UAB_Phi87 | + | 8.8 ± 0.1 | 3.8 ± 0.0 | 3.5 ± 0.1 |
| | - | 8.9 ± 0.0 | 2.4 ± 0.1 | 1.2 ± 0.2 |

Values shown are means ± standard deviations (n = 2). ND, not detected

### Example 6. Lipidic coated bacteriophages stability after lyophilisation

Coated and non-coated bacteriophage UAB_Phi20 was subjected to freeze-drying lyophilisation. The non-coated bacteriophage was set as control for the determination of lyophilisation effect on the bacteriophage titre. The procedure for preparing the lipidic coated bacteriophages was similar to the one above described. A lipid mixture of DLPC, Chol-PEG, Chol and cholesteryl at 1:0.1:0.2:0.7 molar ratios were dissolved in chloroform and the solvent was removed by evaporation under vacuum and nitrogen. The amount of total lipid was 17 mM. The lipids were hydrated with the appropriate solution of each of the bacteriophages containing the predissolved cryoprotective agent (trehalose) at a 1:5 lipid to carbohydrate ratio (3.2 % (w/v)), and the lipid structures thus obtained were homogenized to 400 nm by means of an extruder (Lipex Biomembranes, Vancouver, Canada). After a step of freezing at -80°C for 2 h, the lyophilisation was performed at -40°C for 48 h. Table 4 shows the titre of bacteriophages UAB_Phi20 before and after lyophilisation process.

As it is showed in Table 4 the infectivity of non-coated and coated bacteriophage UAB_Phi20 was 46.8% and 100%, respectively. Therefore, the lipidic coat protects bacteriophages from getting inactivated by the extreme process conditions of the lyophilisation process.

**TABLE 4. Survival of lipidic coated bacteriophages after lyophilisation**

| Bacteriophage | Lipidic coat | Bacteriophage titre (pfu/ml) | | Percentage of infectivity (%) | |
|---|---|---|---|---|---|
| | | Lyophilisation | | | |
| | | - | + | - | + |
| UAB_Phi20 | - | 7.9E+09 | 3.7E+09 | 100 | 46.8 |
| | + | 1.2E+10 | 1.2E+10 | 100 | 100 |

### Example 7. Residence time of lipidic coated bacteriophages in the cecum of broilers.

The experiment was developed in the Servei de Granges i Camps Experimentals of the Universitat Autònoma de Barcelona (Cerdanyola del Vallès, Spain). The area showed a biosecurity level-2 (NBS2). Each poultry yard had a suspended sprue connected to the tap water with continuous refill and a feeder which was adapted depending on the animal needs. Temperature in the room was adapted depending on the growth and metabolism of the chickens, hence during the first week the room was maintained at 30°C and at 8th day the temperature was decreased until 26°C and was maintained during the rest of the experiment. The illumination of the farm alternated cycles of 5 h of green and blue light with cycles of 1 h with blue light, with the purpose to increase the feeding impulse of the animals. Feed and water were supplied ad libitum.

The residence time of bacteriophages in the chick digestive system was determined over 72 h. To achieve this, two groups of 64 one-day chickens were housed on two poultry yards. One group was administered with a non-lipidic coating bacteriophage cocktail and the other with lipidic coating bacteriophage cocktail at a dose of 10¹⁰ pfu of each bacteriophage/animal obtained according to the methods previously described. These cocktails were composed by bacteriophages UAB_Phi20, UAB_Phi78 and UAB_Phi87 (1:1:1) at a concentration of 10¹¹ pfu of each bacteriophage/ml in MgSO₄ 10 mM. One hundred µl was orally administered at 0 h and, twenty one chicks of each group were euthanized at 2, 48 and 72 h and bacteriophages were recovered from cecum of these animals. To do this, cecum homogenates on peptone water were serially diluted and plated onto LB plates using the double agar layered method and were incubated at 37°C for 18 h.

As it is showed in Table 5, after 2 h no significant differences in the recovery of bacteriophages from animals treated with coated and non-coated bacteriophages were observed. However at 48 h lipidic coated and non-coated bacteriophages were found in the cecum of the 90.5 % and 38.1 % of broilers, respectively (P<0.001). Likely, significant differences were found at 72 h. These results demonstrate that the lipidic envelope improves the residence time of bacteriophages in the animals' digestive system.

**TABLE 5. Persistence of coated and non-coated bacteriophages in the ceca of broilers**

| Time (h) | Percentage (%) of broilers with bacteriophages in cecum | |
|---|---|---|
| | Coated | Non-coated |
| 2 | 66.7 | 57.1 |
| 48 | 90.5*** | 38.1 |
| 72 | 38.1 * | 9.5 |

| | | |
|---|---|---|
| *, p < 0.05; *** p < 0.001 | | |

## Claims

**1.** Coated bacteriophages comprising a lipid mixture encasing, enclosing or enveloping one or more bacteriophages and forming a lipidic capsule, vesicle, envelope or liposome-like particle containing one or more bacteriophages, **characterized in that** the lipid mixture comprises:
- a first lipidic component containing at least one lipid substance selected from the group consisting of: 1,2-dilauroyl-rac-glycero-3-phosphocholine (DLPC), 1-palmitoyl-2-oleoyl-sn-glycero-3-phosphocholine (PC), 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC) and 1-palmitoyl-2-oleoyl-sn-glycero-3-phosphocholine (POPC),
- a second lipidic component containing at least one lipid substance selected from the group consisting of: 3β-N-(dimethylaminoethyl) carbamate hydrochloride (cholesteryl), (N-[1-(2,3-Dioleoyloxy)propyl]-N,N,N-trimethylammonium chloride) (DOTAP) and cholesteryl hemisuccinate,
- a third lipidic component containing at least one lipid substance selected from the group consisting of: cholesterol, squalene, ergosterol and phytosterol,
and
- a fourth lipidic component containing at least one lipid substance selected from the group consisting of: cholesteryl-polyethylene glycol 600 sebacate (Chol-PEG600),1,2-distearoyl-*sn*-glycero-3-phosphoethanolamine-N-[amino(polyethylene glycol)-2000 (DSPE- PEG2000), N-(Carbonyl-methoxypolyethyleneglycol 2000)-1,2-dipalmitoyl-sn-glycero-3-phosphoethanolamine (DPPE-PEG5000), 1,2-dipalmitoyl-sn-glycero-3-phosphoethanolamine-N-[methoxy(polyethylene glycol)-350] (PE-PEG350), 1,2-dipalmitoyl-sn-glycero-3-phosphoethanolamine-N-[methoxy(polyethylene glycol)-550] (PE-PEG550), 1,2-dioleoyl-sn-glycero-3-phosphoethanolamine-N-[methoxy(polyethylene glycol)-750] (PE-PEG750), 1,2-dimyristoyl-sn-glycero-3-phosphoethanolamine-N-[methoxy(polyethylene glycol)-1000] (PE-PEG1000), 1,2-distearoyl-sn-glycero-3-phosphoethanolamine-N-[methoxy(polyethylene glycol)-2000] (PE-PEG2000), 1,2-dimyristoyl-sn-glycero-3-phosphoethanolamine-N-[methoxy(polyethylene glycol)-3000] (PE-PEG3000) and 1,2-dioleoyl-sn-glycero-3-phosphoethanolamine-N-[methoxy(polyethylene glycol)-5000] (PE-PEG5000).

**2.** Coated bacteriophages according to claim 1 **characterized in that** the lipid mixture forming the lipidic capsules, vesicles, envelopes or liposome-like particles comprises:
- the first lipidic component in an amount ranging from 40-60%.weight,
- the second lipidic component in an amount ranging from 30-45%.weight,
- the third lipidic component in an amount ranging from 10-40%.weight, and
- the fourth lipidic component in an amount ranging from 0.5-1 0%.weight.

**3.** Coated bacteriophages according to claims 1 - 2, **characterized in that** the lipid mixture forming the lipidic capsules, vesicles, envelopes or liposome-like particles comprises:
- the first lipidic component in an amount of 50%.weight,
- the second lipidic component in an amount of 35%.weight,
- the third lipidic component in an amount of 10% weight, and
- the fourth lipidic component in an amount of 5%.weight.

**4.** Coated bacteriophages according to claims 1 - 3, **characterized in that** the lipid mixture forming the lipidic capsules, vesicles, envelopes or liposome-like particles comprises:
- 50% weight 1,2-dilauroyl-rac-glycero-3-phosphocholine (DLPC),
- 35% cholesteryl 3P-N-(dimethylaminoethyl)carbamate hydrochloride (cholesteryl),
- 10% cholesterol (Chol), and
- 5% cholesteryl-polyethylene glycol 600 sebacate (Chol-PEG600).

**5.** Coated bacteriophages according to claims 1 - 4, **characterized in that** the lipidic capsules, vesicles, envelopes or liposome-like particles are small unilamellar vesicles (SUVs).

**6.** Coated bacteriophages according to claims 1 - 5, **characterized in that** the lipidic capsules, vesicles, envelopes or liposome-like particles have a mean particle size of between 200-600 nm and a net surface charge of +10 / +50 mV.

**7.** Coated bacteriophages according to claims 1 - 6, **characterized in that** the one or more bacteriophages are selected from the group consisting of: tailed bacteriophages from the *Caudovirales* order, UAB-Phi20, deposited in GenBank under accession number GQ422450, (version: GQ422450.1 GI: 321282844), UAB-Phi78 deposited in GenBank under accession number GU595417 (version GU595417.1 GI: 322227495) and UAB_Phi87, deposited in GenBank under accession JN225449, (version JN225449.1 GI: 402760815).

**8.** Coated bacteriophages according to claims 1 - 7, **characterized in that** the one or more bacteriophages are selected from the group consisting of: UAB_Phi20, deposited in GenBank under accession number GQ422450, (version: GQ422450.1 GI: 321282844), UAB_Phi78 deposited in GenBank under accession number GU595417 (version GU595417.1 GI: 322227495) and UAB_Phi87, deposited in GenBank under accession JN225449, (version JN225449.1 GI: 402760815), and **in that** the lipid mixture forming the lipidic capsules, vesicles, envelopes or liposome-like particles is consisting of:
- 50% weight 1,2-dilauroyl-rac-glycero-3-phosphocholine (DLPC),
- 35% cholesteryl 3β-N-(dimethylaminoethyl)carbamate hydrochloride (cholesteryl),
- 10% cholesterol (Chol), and
- 5% cholesteryl-polyethylene glycol 600 sebacate (Chol-PEG600).

**9.** Coated bacteriophages according to claims 1 - 8 **characterized in that** they are lyophilized.

**10.** A process for obtaining coated bacteriophages according to claim 1 - 8, comprising coating the bacteriophages with a lipid mixture by hydrating under agitation an aqueous solution containing the desired bacteriophages, at a concentration up to 10¹¹ pfu/mL, with a dry lipid film having a total amount of lipids from 10 to 30 mM.

**11.** A process for obtaining coated bacteriophages according to claim 9, comprising coating the bacteriophages with a lipid mixture by hydrating under agitation an aqueous solution containing the desired bacteriophages at a concentration up to 10¹¹ pfu/mL,and a cryoprotective agent, with a dry lipid film having a total amount of lipids from 10 to 30 mM.

**12.** A bacteriophage formulation comprising coated bacteriophages according to any of claims 1 - 9 together with acceptable carriers, vehicles, solvents and/or excipients for the prophylactic and therapeutic treatment of bacterial infections in humans and animals, as feed additives or for sanitization and/or sterilization of solid matrices in food production and processing facilities.

**13.** A bacteriophage formulation according to claim 12 **characterized in that** the percentage of coated bacteriophages is comprising between 40% to 55%.

**14.** A bacteriophage formulation according to any of claims 12 and 13 in the form of a suspension, spray, aerosol, powders or granules, emulsions, hard or soft capsules, syrups or elixirs.

**15.** A bacteriophage formulation according to any of claims 12 and 13 in the form of an animal feed, drinking water, sanitizer or sterilization solution.
